# EUROPÄISCHE PATENTSCHRIFT

(11) **EP 1 040 182 B1**
(45) Veröffentlichungstag und Bekanntmachung des Hinweises auf die Patenterteilung: **06.04.2005**
(21) Anmeldenummer: 98929217.2
(22) Anmeldetag: 09.04.1998
(51) Int. Cl.: C12N 1/12

(54) **VERFAHREN ZUR HERSTELLUNG VON BIOMASSE MITTELS PHOTOSYNTHESE**
METHOD FOR PRODUCING BIOMASS BY PHOTOSYNTHESIS
PROCEDE DE PRODUCTION DE BIOMASSE PAR PHOTOSYNTHESE

(30) Priorität: 10.04.1997 DE 19714818; 31.03.1998 DE 19814253
(43) Veröffentlichungstag der Anmeldung: 04.10.2000
(73) Patentinhaber: Bioprodukte Prof. Steinberg GmbH, 06217 Merseburg (DE)
(72) Erfinder: BRONESKE, Jürgen, D-14943 Felgentreu (DE); PULZ, Otto, D-14558 Bergholz-Rehbrücke (DE); FRANKE, Horst, D-14558 Bergholz-Rehbrücke (DE); DÖBEL, Katrin, D-14558 Bergholz-Rehbrücke (DE); OEHLMANN, Hans-Ulrich, D-31141 Hildesheim (DE); UPHOFF, Rainer, D-38122 Braunschweig (DE)
(74) Vertreter: Köckeritz, Günter
(86) Internationale Anmeldenummer: PCT/DE1998/001011
(87) Internationale Veröffentlichungsnummer: WO 1998/045409

(56) Entgegenhaltungen:
- WO-A-96/03494
- JP-A- 52 000 066
- JAVANMARDIAN M. ET AL: "High-density photoautotrophic algal cultures: Design, construction, and operation of a novel photobioreactor system" BIOTECHNOL. BIOENG., 1991, 38/10 (1182-1189), USA, XP000238084
- KISHIMOTO M. ET AL: "CO2 fixation and oil production using *micro*-*algae*" J. FERMENT. BIOENG., 1994, 78/6 (479-482), JAPAN, XP002069173

## Beschreibung

Die Erfindung betrifft ein Verfahren zur Herstellung von Biomasse mittels Photosynthese. Insbesondere betrifft die Erfindung ein Verfahren zur Kultivation von Mikroalgen und ermöglicht eine effektive Produktion von Biomasse durch Fixierung von Kohlendioxid aus CO₂-haltigen Abgasen.

Mikroalgen lassen sich gut kultivieren, und daher sind Techniken zur Nutzung der hohen photosynthetischen Produktivität aus zahlreichen Patenten und Publikationen bekannt (Biotechnology and Bioengineering, Vol.35, 809, 1990, H.Gutermann). Häufig wird das Phytoplankton zur Gewinnung von Wertstoffen oder Pharmazeutika wie Polysacchariden, polyungesättigten Fettsäuren, Farbstoffen, Vitaminen usw. geerntet (Algae and Human Affairs, Cambridge University Press, Cambridge, 1988, K.G.Sprenger et al.). Aber auch als proteinreiche Futterstoffe finden die Biomassen aus Algen Verwendung.

Unter den Verfahren, die zur Erzeugung eines Massenproduktes angewendet werden, sind zunächst die häufig beschriebenen offenen Systeme zu nennen. Hierunter werden raceway ponds, runde Becken und evt. natürliche Lagunen mit einer Wassertiefe von in der Regel 10 - 30 cm verstanden. In diesen Systemen werden die in Suspension vorliegenden Algen mit Hilfe von Rührvorrichtungen wie z. B. Schaufelrädern, Propellern oder Archimedischen Schrauben agitiert, um ein Absetzen der Mikroorganismen zu verhindern. Gleichzeitig gelangen alle Algen durch intensives Mischen in die oberen, von intensiverem Licht durchstrahlten Wasserschichten ( Applied Biochemistry and Biotechnology, Vol.51/52, 681, 1995, H. Matsumoto et al.).

Die Produktivität der Mikroalgen in offenen Systemen hängt stark von der Lichtintensität sowie der Fließgeschwindigkeit ab, die eng mit der Technik der Agitation verbunden ist. Typische Biomasseerträge in offenen Systemen liegen bei 8 - 12 g/m² d.

Eine weitere Steigerung der Biomasseerträge lässt sich durch Verfahren, in denen geschlossene Reaktoren eingesetzt werden, erzielen. Dort lassen sich die Kultivationsbedingungen wie Temperatur, Nährstoffangebot, CO₂-Angebot und Lichtintensität genauer als in offenen Systemen steuern bzw. kontrollieren. Bei den geschlossenen Systemen handelt es sich in der Regel um Platten- oder Röhrenreaktoren, die direkt mit Sonnenlicht bzw. künstlichem Licht bestrahlt werden oder in die das Licht durch Lichtleitsysteme, Spiegel und Linsen eingekoppelt wird (Advanves in Biochemical Engineering/Biotechnology, Vol.46, 63, 1992, I.Karube et al.). Die Biomasseerträge liegen abhängig von der Algenspezies sowie vom Kultivierungsverfahren in den genannten Systemen bei 15 - 25 g/m² d.
Ein derartiges Verfahren wird auch in der Druckschrift "High-density photoautotrophic algal cultures: Design, construction and operation of a novel photobioreactor system" (Biotechnol. Bioeng., 1991, 38/10 (1182-1189), USA, XP000238084) beschrieben.

Da der Kohlenstoffgehalt der Biomasse ca. 60 Massen- % beträgt, wird deutlich, dass CO₂ während der Photosynthese den größten Beitrag zum Stoffaufbau liefert und somit nach preisgünstigen CO₂-Quellen gesucht werden muss. Der CO₂-Gehalt der Atmosphäre ist zu gering, um die erforderlichen Produktivitäten zu gewährleisten. Rauchgas und andere industrielle Abgase haben dagegen das Potential, auch große Algenfarmen mit ausreichenden Mengen an CO₂ zu versorgen. Ein Screening nach Algenspezies, die hohe CO₂-Konzentrationen und die im Abgas enthaltenen Spurengase wie SOₓ, NOₓ und CO tolerieren, ist aus verschiedenen Druckschriften bekannt (Plant Physiol.,82,610, 1986, Y.Marcus et al.; Plant Physiol.91,514, 1989, G.D. Price; Plant Physiol. 94,760, 1990, T.Ogewa). Untersuchungen über das Wachstumsverhalten während der Versorgung der Mikroalgen mit Abgasen zeigten, dass diverse marine Algen, z. B. Tetraselmis suecicca, Nannochloropsis/Phaeodactylum, durch eine Verwendung der Abgase nicht in ihrem Wachstum eingeschränkt wurden und die Kulturen für ein Jahr stabil blieben (Applied Biochemistry and Biotechnology, Vol.51/52, 681, 1995, H. Matsumoto et al.). Diese Ergebnisse können jedoch nicht verallgemeinert werden und sind stark von der verwendeten Spezies und den Kultivationsbedingungen abhängig.
Weiterhin muss die Ernte der Biomasse auf die jeweiligen Mikroorganismen abgestimmt werden, da die entsprechenden Verfahren stark von der Erntekonzentration, der Organismengröße sowie den physiologischen Eigenschaften abhängig sind. Aus der Biotechnologie sowie der Abwassertechnologie sind eine Vielzahl von Verfahren zur Zellernte und Feststoffabtrennung bekannt. Beispielhaft seien Tellerzentrifuge, Lamellenseparator und Filtration mit Flockungsmitteln genannt ("Micro - algal Technology", Cambridge University Press, 1988, M.A.Borowitzka, L.J.Borowitzka (eds.)).

Gleiches wie für die Ernte gilt auch für den Aufschluss sowie die Extraktion von Mikroalgen. Die zum Einsatz kommenden Verfahren müssen auf die Zelle und den Verwendungszweck der Biomasse abgestimmt werden.

Der vorliegenden Erfindung liegt die Aufgabe zugrunde, ein Verfahren zur Herstellung von Biomasse mittels Photosynthese, insbesondere zur Kultivation von Mikroalgen vorzustellen, welches eine effektive Produktion von Biomasse durch Fixierung von Kohlendioxid aus CO₂-haltigen Abgasen unter Vermeidung der Nachteile bekannter Verfahren ermöglicht. Mit der vorliegenden Erfindung sollen bekannte derartige Verfahren hinsichtlich ihrer Energiebilanz, ihres Herstellungsaufwandes und einer optimalen Ausnutzung der zur Verfügung stehenden Lichtenergie dahingehend verbessert werden, dass diese für einen großtechnischen Einsatz geeignet sind.

Erfindungsgemäß wird diese Aufgabe durch die Merkmale des Anspruchs 1 gelöst. Vorteilhafte Ausbildungen des Verfahrens sind in den Ansprüchen 2 bis 22 enthalten. Die Erfindung beinhaltet auch die Verwendung besonders bevorzugter Algenspezies.

Nach der vorliegenden Erfindung erfolgt die Herstellung von Biomasse mittels Photosynthese, wobei durch Beimpfen einer Nährlösung mit Mikroorganismen eine Kultursuspension hergestellt wird und die Kultursuspension in einem Gaswäscher mit einem CO₂-enthaltenden Gas unter Sättigung der im Gas enthaltenen Komponenten bis zur Blasenfreiheit konditioniert wird. Dabei wird in der Kultursuspension ein pH-Wert von > 5,0 und < 7,0 eingestellt; wodurch die Mikroorganismen einem Konditionierungsstress (pH-Stress) unterliegen, wodurch der Fettanteil der Biomasse insgesamt sowie der Anteil spezieller Fettsäuren wie beispielsweise der Anteil mehrfach ungesättigter Fettsäure überraschend gesteigert werden kann. Die quantitative Zusammensetzung der Zellinhaltsstoffe kann dabei gezielt modifiziert werden, wobei ein Fettgehalt von mehr als 20 % in der Trockenbiomasse erreicht wird. Weiterhin erfolgt während der logarithmischen, bis weit in die stationäre Wachstumsphase der Mikroorganismen eine Zuführung weiterer organischer Kohlenstoffquellen. Die Suspension wird anschließend unter Erzeugung eines gleichmäßigen Strömungsprofils in einen Reaktor geleitet, in dem bei Belichtung und Einstellung einer optimalen Fließgeschwindigkeit der Lösung eine Abreicherung des in der Kultursuspension enthaltenen CO₂ durch Aufbau energiereicher Kohlenstoffverbindungen und Bildung weiterer Stoffwechselprodukte erfolgt. Die Suspension wird anschließend zum Gaswäscher zurückgeführt und zirkuliert in der Reaktoranlage. Die im Reaktor erzeugte Biomasse wird anschließend aus der Kultursuspension abgetrennt. Das gleichmäßige Strömungsprofil der konditionierten Kultursuspension wird erfindungsgemäß dadurch erzeugt, dass diese in einen Reaktor geleitet wird, der ein photosynthetisch und hydrodynamisch aktives Dünnschichtsystem umfasst.

Das Dünnschichtsystem besteht dabei aus mindestens einem Modul, wobei das Modul aus einer Vielzahl von zueinander beabstandeten, übereinander liegenden und parallel zueinander verlaufenden Dünnschichtgefäßen besteht, deren Eintrittsund Austrittsseiten über Anschlussstücke und Verbinder mit einem Modulverlauf beziehungsweise Rücklauf verbunden sind. Die einzelnen Verbinder weisen dabei jeweils eine Querschnittsfläche auf, die ein gleichmäßiges Strömungsprofil über alle Glasrohre ermöglicht. Dabei nimmt die Querschnittsfläche der Verbinder in Fließrichtung des Modulvorlaufes zu, während die Querschnittsfläche der Verbinder des Modulrücklaufes in Fließrichtung abnimmt. Modulvor- und -rücklauf bestehen aus jeweils aus einem Rohr mit Anschlussstücken für die Verbinder, wobei die Querschnittsfläche des Modulvorlaufes in Fließrichtung abnimmt und die Querschnittsfläche des Modulrücklaufes in Fließrichtung zunimmt. Durch die querschnittsreduzierten beziehungsweise -erweiterten Verbinder und querschnittsreduzierten beziehungsweise -erweiterten Modulvor- und -rückläufe wird ein gleichmäßiges Strömungsprofil über alle Dünnschichtgefäße ermöglicht.

Nach einem weiteren Merkmal der Erfindung wird das gleichmäßige Strömungsprofil der konditionierten Kultursuspension dadurch erzeugt, dass diese in einen Reaktor eingeleitet wird, der aus mindestens einem einstückig extrudierten transparenten Plattenmodul besteht, welches eine Vielzahl von übereinander liegenden und parallel zueinander verlaufenden durchgehenden Kanälen aufweist, deren Eintritts- und Austrittsseiten in einen Flüssigkeitssammler und/oder Flüssigkeitsverteiler münden, wobei der Flüssigkeitsverteiler in Fließrichtung eine Querschnittsreduzierung und der Flüssigkeitssammler eine Querschnittserweiterung aufweist. Dadurch kann erfindungsgemäß über alle Kanäle eine optimale, gleichmäßige Fließgeschwindigkeit der Lösung im Reaktor eingestellt werden. Es wird mit überraschend einfachen Mitteln erreicht, dass mit geringen Strömungsgeschwindigkeiten turbulente Verhältnisse im Kulturmedium erhalten werden, die für eine optimale Lichtenergieausnutzung erforderlich sind. Darüber hinaus kann der Strömungswiderstand im Vergleich zu den bekannten Lösungen erheblich verringert werden.

Nach einem bevorzugten Merkmal der Erfindung werden als weitere C-Quellen der Kultursuspension Einfach- und/oder Mehrfachzucker, und zwar insbesondere Glucose, in einer Konzentration von 0,3 bis 10 g/l Kultursuspension zugegeben.

Durch diese mixotrophe Ernährungsweise der Mikroorganismen wird das Potential, der Algenzelle Kohlenstoff auf verschiedenen Stoffwechselwegen mit Hilfe unterschiedlicher biochemischer Prozesse für die Bildung von zelleigener, energiereicher Substanz zu assimilieren, voll ausgeschöpft. Außerdem können die Prozesse der Photorespiration, durch die 1 bis 10 % des neu assimilierten Kohlenstoffes verloren gehen, minimiert werden.

Mit der vorliegenden Erfindung wird ein Verfahren vorgestellt, welches sich gegenüber bislang bekannten Verfahren durch ein reduziertes Kontaminationsrisiko, reproduzierbare Kultivationsbedingungen, hohe Flexibilität in Bezug auf Umwelteinflüsse, geringeren Platzbedarf und reproduzierbare Biomassequalitäten auszeichnet.

Nach einem besonderen Merkmal der Erfindung handelt es sich bei dem Stoffwechselprodukt um ein O₂-haltiges Gas, welches durch Phasentrennung nach Durchgang durch den Reaktor abgetrennt wird und einer weiteren Verwendung zugeführt werden kann. Diese Sauerstoffproduktion macht das erfindungsgemäße Verfahren besonders effizient.

Nach einem bevorzugten Merkmal der Erfindung werden als Mikroorganismen phototrophe Organismen und/oder Zellkulturen, insbesondere Mikroalgen eingesetzt. Es wurde gefunden, dass mit den Mikroalgenspecies Chlorella vulgaris und/oder Scenedesmus spp./Microcystis (Mischkultur) sich besonders gut die oben beschriebenen Vorteile des Verfahrens erreichen lassen.

Nach einem weiteren Merkmal der Erfindung werden nach dem vorliegenden Verfahren Algenbiomasse und deren Stoffwechselprodukte erzeugt, wobei als Nährlösung Wasser und Stoffe, die für den Aufbau energiereicher Kohlenstoffverbindungen erforderlich sind, eingesetzt werden. Dabei handelt es sich um Stickstoffverbindungen, Phosphate, Kalium-, Calcium-, Magnesiumverbindungen und Spurenelemente. Nach einem anderen Merkmal der Erfindung enthält die Nährlösung auch organische Substanzen, z. B. Kohlenhydrate wie Zucker und/oder Acetate und/oder puffernde Substanzen wie Tris und/oder Borax und/oder Kalk.

Nach einem besonderen Merkmal der Erfindung kann die Nährlösung auch nährstoffhaltige Abwässer, einschließlich Klärschlämme und/oder synthetische Lösungen enthalten. Dadurch wird ein äußerst effektiver Einsatz derartiger Abwässer ermöglicht.

Ein ebenfalls bevorzugtes Merkmal der Erfindung beinhaltet, dass als CO₂enthaltendes Gas ein Abgas, und zwar insbesondere ein Abgas aus Verbrennungsprozessen, Kalkbrennprozessen und /oder metallurgischen Prozessen eingesetzt wird. Damit müssen derartige Abgase nicht mehr an die Umwelt abgegeben werden, sondern können einer sinnvollen Verwendung zugeführt werden. Die Algenspezies erhalten darüber hinaus ausreichende Mengen an CO₂, um die erforderliche Produktivität zu gewährleisten.

Die Konditionierung der Kultursuspension mit dem CO₂-enthaltenden Gas unter Sättigung der im Gas enthaltenen Komponenten erfolgt wie beschrieben in einem Gaswäscher. Die Komponenten des Gases lösen sich dabei in der Kultursuspension bis zur Sättigung und Komponenten der Kultursuspension, deren Konzentration die Gaslöslichkeit in der Kultursuspension überschreitet, werden ausgetragen. Die Suspension verlässt den Gaswäscher blasenfrei. Mit dem Gaswäscher ist ein geregelter CO₂-Eintrag bei geringen CO₂-Verlusten möglich.

Ein bevorzugtes Merkmal der Erfindung beinhaltet, dass das aus Verbrennungsprozessen stammende CO₂-enthaltende Abgas in einem Abgaskühler temperiert wird und das dabei entstehende Kondensat mit dem CO₂-enthaltende Abgas über einen Gaswäscher in den Reaktor geleitet wird. Das Kondensat wird damit Bestandteil der Kultursuspension und einer sinnvollen Verwendung zugeführt.

Letztendlich wird dadurch die erforderliche Pumpleistung wesentlich reduziert, wodurch die Energiebilanz besonders vorteilhaft verbessert wird. Die Verbindung mehrerer Plattenmodule ermöglicht außerdem den Aufbau beliebig großer Reaktoren für den großtechnischen Einsatz aus einfachen Standardbauteilen.

Nachfolgend soll die Erfindung anhand eines Ausführungsbeispieles näher erläutert werden.

In der Abbildung zeigt die
- Figur 1:: eine beispielhafte Ausführungsform des Reaktors und den schematischen Ablauf des Verfahrens,
- Figur 2:: eine weitere Variante eines Reaktors mit Dünnschichtsystemen.

Aus dem Brennprozess eines Kalkbrennofens wird ein Abgas 27a mit einer CO₂-Konzentration von ca. 25 % einem Abgaskühler (nicht dargestellt) zugeführt. Im Abgaskühler wird das CO₂-enthaltende Abgas auf eine Temperatur von ca. 35 °C temperiert. Während des Kühlprozesses entsteht ein Kondensat.

Das CO₂-enthaltende Abgas und das Kondensat werden über einen Gaswäscher 5 in einen Reaktor geleitet, wobei im Gaswäscher 5 eine Kultursuspension 4a mit dem CO₂-enthaltenden Abgas 27a und dem Kondensat (nicht dargestellt) unter Sättigung der im Abgas enthaltenen Komponenten bis zur Blasenfreiheit konditioniert wird.
Die Kultursuspension 4a besteht aus einer Nährlösung und einer Mikroalgenspecies Chlorella vulgaris. Die Nährlösung besteht aus Wasser 9, anorganischen Nährstoffen und Tris(hydroxymethyl)-aminomethan.

Das Wasser wird dabei als Abwasser aus dem Kalkbrennofen zur Verfügung gestellt. Im Abwasser sind in geringer Konzentration anorganische Nährstoffe gelöst.
Die Nährlösung (NL) beinhaltet dabei folgende anorganische Nährstoffe.

| **Komponente** | **Einsatzmenge (g/l)** |
|---|---|
| *Makrobestandteile* | |
| Harnstoff | 0,3 |
| Ammoniumnitrat | 0,4 |
| Kaliumdihydrogenphosphat | 0,34 |
| Magnesiumsulfat | 0,5 |
| Eisensulfat | 0,5 |

| *Mikrobestandteile I pro Liter Stammlösung* (*0,01 ml* /*I NL*) | |
|---|---|
| Zinksulfat | 74,0 |
| Borax | 5,7 |
| Kobaltsulfat | 23,8 |
| Kupfersulfat | 23,6 |
| Mangansulfat | 410 |

| *Mikrobestandteile II pro Liter Stammlösung ( 0, 0 1 ml* / *I NL)* | |
|---|---|
| Ammoniummolybdat | 9,2 |

Tris(hydroxymethyl)-aminomethan ist eine organische Substanz, die als Säurepuffer der Nährlösung in einer Konzentration von ca. 1,0 g/l zugesetzt wird.

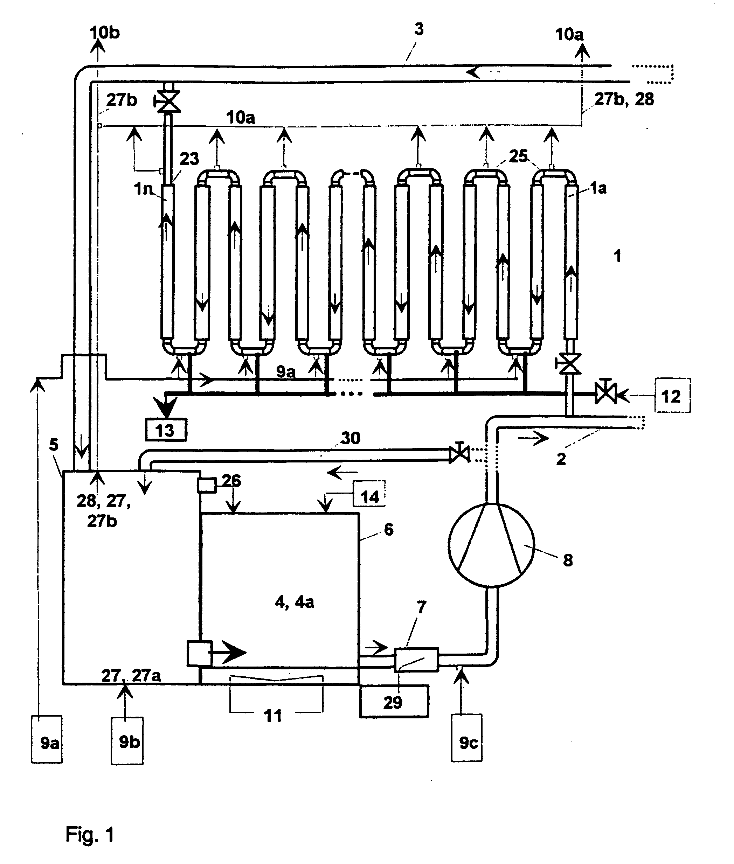

Die konditionierte Kultursuspension zirkuliert im Reaktor welcher gemäß der Figur 1 aus einer Plattenmodulanlage 1 aus einstückig extrudierten transparenten Plattenmodulen 1a...1n, einem Ausgleichsbehälter 6, einer Systempumpe 8, einer Messstrecke 7, einem Vorlauf 2, einem Rücklauf 3 und dem Gaswäscher 5 besteht und wird mit Hilfe der natürlichen Sonneneinstrahlung 12 belichtet. Die konditionierte Kultursuspension gelangt aus dem Ausgleichsbehälter 6 durch die Systempumpe 8 über den Vorlauf 2 in das Plattenmodul 1a.
Das Plattenmodul 1a weist eine Vielzahl von übereinander liegenden und parallel zueinander laufenden durchgehenden Kanälen auf, deren Eintritts- und Austrittsseiten in einem Flüssigkeitssammler 21 bzw. Flüssigkeitsverteiler 15 münden.
Der Flüssigkeitsverteiler 15 besitzt dabei in Fließrichtung eine Querschnittsreduzierung 19 und der Flüssigkeitssammler 21 besitzt eine Querschnittserweiterung 22.
Dadurch wird im Plattenmodul ein gleichmäßiges Strömungsprofil der konditionierten Kultursuspension gewährleistet.

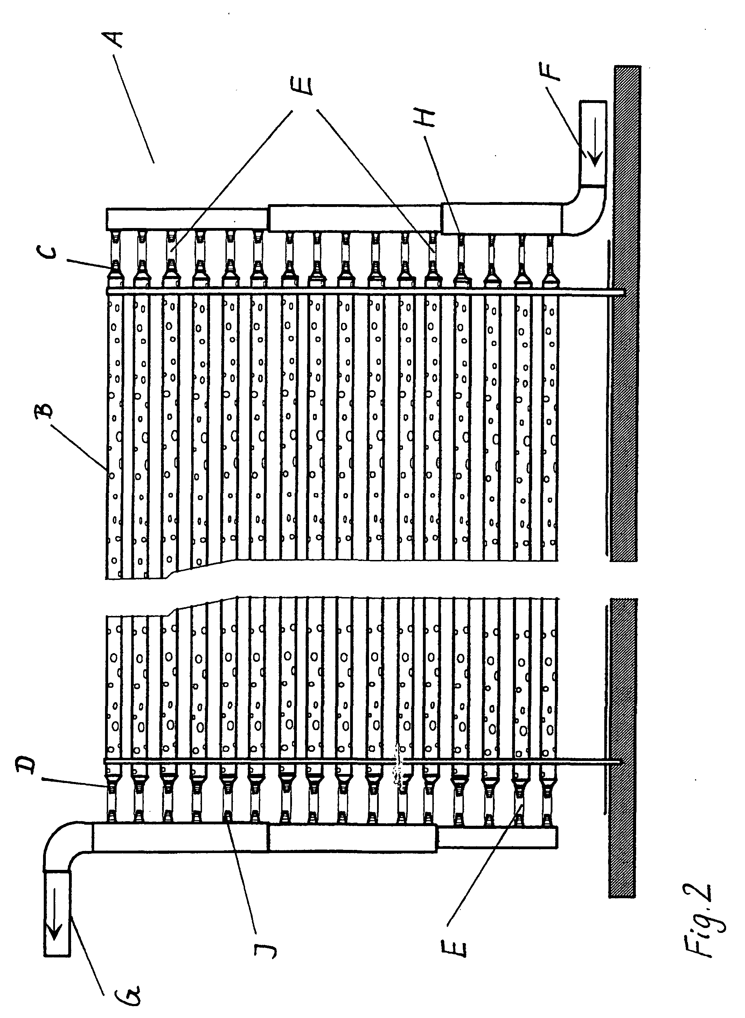

Die gleiche Wirkung wird selbstverständlich auch in einem Reaktor nach Figur 2 erreicht. Dieser Reaktor besteht aus einem photosynthetisch und hydrodynamisch aktiven Dünnschichtsystem aus mindestens einem Modul A mit einer Vielzahl von zueinander beabstandeten, übereinander liegenden und parallel zueinander verlaufenden, röhrenförmigen Dünnschichtgefäßen B, deren Eintritts- und Austrittsseiten über Anschlussstücke C, D und Verbinder E für die übereinander liegenden Dünnschichtgefäße jeweils eine Querschnittsfläche aufweisen, die in Fließrichtung des Modulvorlaufes F zunimmt und in Fließrichtung des Modulrücklaufes G abnimmt. Dabei bestehen Modulvor- und -rücklauf F und G jeweils aus einem Rohr mit Anschlussstücken H und I für die Verbinder E, wobei die Querschnittsfläche des Modulvorlaufes F in Fließrichtung abnimmt und die Querschnittsfläche des Modulrücklaufes G in Fließrichtung zunimmt.

Im Plattenmodul erfolgen das Biomassewachstum, die Abreicherung des gelösten CO₂ und die Anreicherung von Sauerstoff 28.
Nach dem Plattenmodul gelangt die Kultursuspension 4a über den Rücklauf 3 und den Gaswäscher 5 in den Ausgleichsbehälter 6 zurück.

Das CO₂-enthaltende Abgas 27a wird mit Hilfe des Gaswäschers 5 in der Kultursuspension 4a gelöst, und der im Verlauf der photobiologischen Prozesse gebildete Sauerstoff 28 mit den nicht genutzten Komponenten des Abgases 27b wird ausgetragen.

Die Konditionierung des CO₂-enthaltende Abgases erfolgt in Abhängigkeit der pH - und Temperatur- und Optischen Dichtewerten der Kultursuspension, welche in der Messstrecke 7 vor der Systempumpe 8 erfasst werden.
Die Messung, Steuerung, Regelung und Speicherung von Zustandsgrößen der Kultursuspension und des CO₂-enthaltenden Abgases erfolgt dabei über eine zentrale Mess-, Steuer-, Regel- und Speichereinheit 29.

Die Trockenmassekonzentration der Mikroalgenspecies Chlorella vulgaris in der Kultursuspension beträgt ca. 2 g Trockensubstanz/l. Im Verlaufe des Wachstums der Biomasse werden energiereiche Kohlenstoffverbindungen aufgebaut. Die Biomasseemte erfolgt bei einer Trockenmassekonzentration in der Kultursuspension von > 2 g/l.
Mit Hilfe der Systempumpe 8 wird die Kultursuspension einer Emteeinrichtung (nicht dargestellt) zugeführt.
Die Erntevorrichtung besteht aus einem Zwischenspeicher, einer Förderpumpe und einer Zentrifuge. Die Zentrifuge trennt die Biomasse von der Nährlösung, welche wieder in den Reaktor zurückgeführt wird. Die Biomasse besitzt eine Trockenmassekonzentration von ca. 120 g/l und ist dementsprechend feststoffreich, aber pumpfähig.

Die somit erhaltene Biomasse wird einer Verarbeitungseinrichtung (nicht dargestellt) zugeführt. Diese Verarbeitungseinrichtung ist eine Einrichtung, die auf extraktiver Basis arbeitet. Die Biomasse wird einer Lösungsmittelextraktion unterzogen, wobei die energiereichen Kohlenstoffverbindungen als Energieträger gewonnen werden.

In einem weiteren Verfahrensschritt (nicht dargestellt) werden die gewonnen energiereichen Kohlenstoffverbindungen einer Veresterung unterworfen und somit ein Biodiesel hergestellt. Bis zum Zeitpunkt des Verbrauchs wird der Biodiesel in einem Speicher zwischengelagert.

Nach einem weiteren Beispiel wird die erhaltene Biomasse als Futtermittelbeziehungsweise Futtermittelzusatz eingesetzt.
Durch das erfindungsgemäße Verfahren wird eine Modifizierung der quantitativen Zusammensetzung der Zellinhaltsstoffe ermöglicht. Als Ausführungsbeispiel gilt die wesentliche Erhöhung der Fettgehalte auf vorzugsweise mehr als 20 % in der Trockenbiomasse. Der Zusatz von ca. 1 % der erfindungsgemäßen Algenbiomasse zum Gesamtfutter bei Legehennen bringt überraschende Ergebnisse. Die höheren Legeleistungen bei Legehennen, sowohl in ökologischer als auch konventioneller Haltung, werden auf die gesundheitsfördernden synergistischen Wirkungen der natürlichen, biologisch gebundenen, ausgewogenen, bioaktiven Zellinhalts-Wirkstoffe der Mikroalge Chlorella vulgaris, wie Ballaststoffe, insbesondere lösliche, resistente Stärke, Oligosaccharide, Plysaccharide, Vitamine, essentielle mehrfach ungesättigte Fettsäuren, essentielle Aminosäuren, Mineralien und Spurenelemente zurückgeführt.
Wirkungen dieser Stoffkomplexe sind die Förderung der Darm-Mikroflora und die Bildung von immunbiologisch und -physiologisch aktiven Substanzen, die Senkung des Blutzucker-, Cholesterinspiegels und des Bluthochdrucks, antitumorale und atimutagene Effekte.

## Patentansprüche

1. Verfahren zur Herstellung von Biomasse mittels Photosynthese, wobei durch Beimpfen einer Nährlösung mit Mikroorganismen eine Kultursuspension hergestellt wird,
- die Kultursuspension in einem Gaswäscher mit einem CO₂-enthaltenden Gas unter Sättigung der im Gas enthaltenen Komponenten bis zur Blasenfreiheit konditioniert wird, wobei in der Kultursuspension ein pH-Wert von >5,0 und <7,0 eingestellt wird und der Kultursuspension während der logarithmischen bis weit in die stationäre Wachstumsphase der Mikroorganismen weitere organische Kohlenstoff-Quellen zugeführt werden,
- die Suspension unter Erzeugung eines gleichmäßigen Strömungsprofils in einen Reaktor geleitet wird, in dem bei Belichtung und Einstellung einer optimalen Fließgeschwindigkeit eine Abreicherung des in der Kultursuspension enthaltenen CO₂ durch Aufbau energiereicher Kohlenstoffverbindungen und Bildung weiterer Stoffwechselprodukte erfolgt,
- die Suspension zum Gaswäscher rückgeführt wird und in der Reaktoranlage zirkuliert,
- die im Reaktor erzeugte Biomasse aus der Kultursuspension abgetrennt wird, **dadurch gekennzeichnet, dass** das gleichmäßige Strömungsprofil der konditionierten Kultursuspension erzeugt wird, indem diese in einen Reaktor eingeleitet wird, der ein photosynthetisch und hydrodynamisch aktives Dünnschichtsystem aus mindestens einem Modul (A) mit einer Vielzahl von zueinander beabstandeten, übereinanderliegenden und parallel zueinander verlaufenden, röhrenförmigen Dünnschichtgefäßen (B) besteht, deren Eintrittsund Austrittsseiten über Anschlussstücke (C, D) und Verbinder (E) mit einem Modulvorlauf (F) beziehungsweise Rücklauf (G) verbunden sind, wobei die einzelnen Verbinder (E) für die übereinanderliegenden Dünnschichtgefäße (B) jeweils eine Querschnittsfläche aufweisen, die in Fließrichtung des Modulvorlaufes (F) zunimmt und in Fließrichtung des Modulrücklaufes (G) abnimmt und Modulvor- und -rücklauf (F, G) jeweils aus einem Rohr mit Anschlussstücken (H, I) für die Verbinder (E) bestehen, wobei die Querschnittsfläche des Modulvorlaufs (F) in Fließrichtung abnimmt und die Querschnittsfläche des Modulrücklaufes (G) in Fließrichtung zunimmt.

2. Verfahren nach Anspruch 1, **dadurch gekennzeichnet, dass** das gleichmäßige Strömungsprofil der konditionierten Kultursuspension erzeugt wird, indem diese in einen Reaktor eingeleitet wird, der aus mindestens einem einstückig extrudierten transparenten Plattenmodul besteht, welches eine Vielzahl von übereinanderliegenden und parallel zueinander verlaufenden durchgehenden Kanälen aufweist, deren Eintritts- und Austrittsseiten in einen Flüssigkeitssammler und/oder Flüssigkeitsverteiler münden, wobei der in Fließrichtung gesehene Flüssigkeitsverteiler eine Querschnittsreduzierung und der Flüssigkeitssammler eine Querschnittserweiterung aufweist, wodurch über alle Kanäle eine optimale, gleichmäßige Fließgeschwindigkeit der Suspension im Reaktor eingestellt wird.

3. Verfahren nach den Ansprüchen 1 bis 2, **dadurch gekennzeichnet, dass** als weitere organische Kohlenstoffquellen der Kultursuspension Einfach- und/oder Mehrfachzucker zugegeben werden.

4. Verfahren nach Anspruch 3, **dadurch gekennzeichnet, dass** als weitere organische Kohlenstoffquellen der Kultursuspension Glucose in einer Konzentration von 0,3 bis 10 g/l Kultursuspension zugegeben wird.

5. Verfahren nach einem der obigen Ansprüche, **dadurch gekennzeichnet, dass** die quantitative Zusammensetzung der Zellinhaltsstoffe der Biomasse modifiziert wird, wobei ein Fettgehalt von mehr als 20 % in der Trockenbiomasse erreicht wird.

6. Verfahren nach einem der obigen Ansprüche, **dadurch gekennzeichnet, dass** das Stoffwechselprodukt ein O₂-haltiges Gas ist, welches durch Phasentrennung nach Durchgang durch den Reaktor abgetrennt wird.

7. Verfahren nach einem der obigen Ansprüche, **dadurch gekennzeichnet, dass** die Abtrennung der Biomasse unter Ausnutzung von Dichteunterschieden in Absetzbecken und/oder Zentrifugen und/oder unter Ausnutzung der Teilchengröße in Filtereinrichtungen erfolgt und die Kulturlösung in das Reaktorsystem zurückgeführt wird.

8. Verfahren nach einem der obigen Ansprüche, **dadurch gekennzeichnet, dass** Algenbiomasse und deren Stoffwechselprodukte erzeugt werden und als Nährlösung Wasser und Stoffe, die für den Aufbau energiereicher Kohlenstoffverbindungen erforderlich sind, eingesetzt werden.

9. Verfahren nach Anspruch 8, **dadurch gekennzeichnet, dass** die Nährlösung Stickstoffverbindungen, Phosphate, Kalium-, Calcium, Magnesiumverbindungen und Spurenelemente enthält.

10. Verfahren nach den Ansprüchen 8 und 9, **dadurch gekennzeichnet, dass** die Nährlösung als weitere organische Kohlenstoffquellen Zucker und/oder Acetate und/oder als puffernde Substanzen Tris(hydroxymethyl-aminomethan) und/oder Borax und/oder Kalk enthält.

11. Verfahren nach den Ansprüchen 8 bis 10, **dadurch gekennzeichnet, dass** als Nährlösung nährstoffhaltige Abwässer, einschließlich Klärschlämme und/oder synthetische Lösungen verwendet werden.

12. Verfahren nach einem der obigen Ansprüche, **dadurch gekennzeichnet, dass** als Mikroorganismen phototrophe Organismen und/oder Zellkulturen eingesetzt werden.

13. Verfahren nach Anspruch 12, **dadurch gekennzeichnet, dass** als Mikroorganismen Mikroalgen eingesetzt werden.

14. Verfahren nach Anspruch 12 und 13, **dadurch gekennzeichnet, dass** als Mikroorganismen die Mikroalgenspecies Chlorella ssp. und/oder Scenedesmus spp. und/oder Microcystis ssp. als Rein- oder Mischkulturen eingesetzt werden.

15. Verfahren nach einem der obigen Ansprüche, **dadurch gekennzeichnet, dass** als CO₂-enthaltendes Gas ein Abgas eingesetzt wird.

16. Verfahren nach Anspruch 15, **dadurch gekennzeichnet, dass** als CO₂enthaltendes Gas ein Abgas aus Verbrennungsprozessen, Kalkbrennprozessen und/oder metallurgischen Prozessen eingesetzt wird.

17. Verfahren nach einem der obigen Ansprüche, **dadurch gekennzeichnet, dass** das CO₂-enthaltende Abgas in einem Abgaskühler temperiert wird und das dabei entstehende Kondensat mit dem CO₂-enthaltenden Abgas über einen Gaswäscher in den Reaktor geleitet wird.

18. Verfahren nach einem der obigen Ansprüche, **dadurch gekennzeichnet, dass** das Gas im Gegenstrom zur Kultursuspension durch den Gaswäscher geführt wird.

19. Verfahren nach einem der obigen Ansprüche, **dadurch gekennzeichnet, dass** die Belichtung der im Reaktor befindlichen Kultursuspension durch natürliche direkte und/oder diffuse Sonneneinstrahlung und/oder künstliche Beleuchtung erfolgt.

20. Verwendung der nach einem der obigen Ansprüche erzeugten Biomasse zur Weiterverarbeitung als Energieträger.

21. Verwendung der nach einem der obigen Ansprüche erzeugten Biomasse zur Herstellung von chemischen und/oder pharmazeutischen Grund- und Wirkstoffen.

22. Verwendung der nach einem der obigen Ansprüche hergestellten Biomasse als Futtermittel- und/oder Futtermittelzusatz.

## Claims

1. Method for producing biomass by means of photosynthesis, in which a culture suspension is produced by inoculating a nutrient solution with micro-organisms,
- the culture suspension is conditioned in a gas washer with a CO₂-containing gas, the components contained in the gas being saturated until the suspension is bubble-free, a pH value of >5.0 and <7.0 being set in the culture suspension and additional organic carbon sources being supplied to the culture suspension during the logarithmic growth phase of the micro-organisms until far into their stationary growth phase,
- the suspension is led, with the generation of a uniform flow pattern, into a reactor in which, when the suspension is exposed and an optimum flow speed is set, depletion of the CO₂ contained in the culture suspension takes place due to the build-up of high-energy carbon compounds and the formation of other metabolic products,
- the suspension is returned to the gas washer and circulated in the reactor system,
- the biomass produced in the reactor is separated from the culture suspension, **characterised in that** the uniform flow pattern of the conditioned culture suspension is generated by its being introduced into a reactor having a thin-film system which is active in terms of photosynthesis and hydrodynamics and comprises at least one module (A) having a large number of tubular thin-film vessels (B). spaced apart from one another, lying the one above the other and extending parallel to one another, the entry and exit sides of which are connected via connecting pieces (C,D) and connectors (E) to a module forward flow (F) or respectively return flow (G), the individual connectors (E) for the thin-film vessels (B) lying the one above the other each having a cross-sectional area which increases in the flow direction of the module forward flow (F) and decreases in the flow direction of the module return flow (G) and the module forward flow and return flow (F, G) each comprising a pipe with connecting pieces (H, I) for the connectors (E), the cross-sectional area of the module forward flow (F) decreasing in the flow direction and the cross-sectional area of the module return flow (G) increasing in the flow direction.

2. Method according to claim 1, **characterised in that** the uniform flow pattern of the conditioned culture suspension is generated by the latter being introduced into a reactor which comprises at least one transparent plate module extruded as one piece and which has a large number of continuous channels lying the one above the other and extending parallel to one another, the entry and exit sides of which open out into a liquid collector and/or liquid distributor, the liquid distributor having a cross-sectional reduction, when viewed in the flow direction, and the liquid collector having a cross-sectional widening, by means of which an optimum uniform flow speed of the suspension in the reactor is set over all the channels.

3. Method according to claims 1 to 2, **characterised in that** simple and/or composite sugars are added to the culture suspension as additional organic carbon sources.

4. Method according to claim 3, **characterised in that** glucose is added to the culture suspension as an additional organic carbon source in a concentration of 0.3 to 10 g/l culture suspension.

5. Method according to one of the above claims, **characterised in that** the quantitative composition of the cell components of the biomass is modified, a fat content of more than 20 % being achieved in the dry biomass.

6. Method according to one of the above claims, **characterised in that** the metabolic product is an O₂-containing gas which is separated by phase separation after it has passed through the reactor.

7. Method according to one of the above claims, **characterised in that** the biomass is separated by exploiting density differences in the settling tank and/or centrifuging and/or by exploiting the particle size in filter devices, and the culture solution is led back into the reactor system.

8. Method according to one of the above claims, **characterised in that** algae biomass and its metabolic products are produced, and water and substances which are necessary for building up high-energy carbon compounds are used as the nutrient solution.

9. Method according to claim 8, **characterised in that** the nutrient solution contains nitrogen compounds, phosphates, potassium compounds, calcium compounds and magnesium compounds and trace elements.

10. Method according to claims 8 and 9, **characterised in that** the nutrient solution contains, as additional organic carbon sources, sugars and/or acetates, and/or as buffering substances, tris(hydroxymethylaminomethane) and/or borax and/or lime.

11. Method according to claims 8 to 10, **characterised in that** waste waters containing nutrients, including sewage sludge and/or synthetic solutions, are used as the nutrient solution.

12. Method according to one of the above claims, **characterised in that** phototropic organisms and/or cell cultures are used as micro-organisms.

13. Method according to claim 12, **characterised in that** micro-algae are used as micro-organisms.

14. Method according to claims 12 and 13, **characterised in that** as the micro-organisms, the micro-algae species chlorella ssp. and/or scenedesmus spp. and/or microcystis ssp. are used as pure or mixed cultures.

15. Method according to one of the above claims, **characterised in that** a waste gas is used as the CO₂-containing gas.

16. Method according to claim 15, **characterised in that** a waste gas from combustion processes, lime-burning processes and/or metallurgical processes is used as the CO₂-containing gas.

17. Method according to one of the above claims, **characterised in that** the temperature of the CO₂-containing waste gas is controlled in a waste-gas cooler and the condensate thereby produced is led with the CO₂-containing waste gas into the reactor via a gas washer.

18. Method according to one of the above claims, **characterised in that** the gas is guided through the gas washer against the flow of the culture suspension.

19. Method according to one of the above claims, **characterised in that** the exposure of the culture suspension located in the reactor takes place through natural direct and/or diffuse solar radiation and/or artificial illumination.

20. Use of the biomass generated according to one of the above claims for further processing as a fuel.

21. Use of the biomass generated according to one of the above claims for the production of basic and active chemical and/or pharmaceutical substances.

22. Use of the biomass produced according to one of the above claims as an animal feedstuff and/or feedstuff additive.

## Revendications

1. Procédé de production de biomasse par photosynthèse, où l'on produit une suspension de culture par inoculation d'une solution nutritive avec des micro-organismes,
- la suspension de culture est conditionnée dans un laveur de gaz avec un gaz contenant du CO₂ sous saturation des composants présents dans le gaz jusqu'à liberté de soufflage, un pH > 5,0 et < 7,0 étant ajusté dans la suspension de culture et d'autres sources organiques de carbone étant amenées à la suspension de culture pendant la phase de croissance logarithmique des micro-organismes jusqu'à loin dans la phase de croissance stationnaire,
- la suspension est conduite, en produisant un profil de circulation régulier, dans un réacteur, où un appauvrissement du CO₂ obtenu dans la suspension de culture s'effectue par constitution de composés de carbone riches en énergie et formation d'autres produits métaboliques, tout en fournissant un éclairage et en ajustant une vitesse de flux optimale,
- la suspension est renvoyée au laveur de gaz et circule dans l'installation de réacteur,
- la biomasse produite dans le réacteur à partir de la suspension de culture est séparée,
**caractérisé en ce que** le profil de circulation régulier de la suspension de culture conditionnée est produit en introduisant celle-ci dans un réacteur qui est un système en couches minces, actif du point de vue hydrodynamique et photosynthétique, constitué d'au moins un module (A) ayant une multitude de réservoirs en couches minces (B) tubulaires, parallèles et superposés, situés à une certaine distance les uns des autres, dont les côtés entrée et sortie sont reliés par le biais de pièces de branchement (C, D) et de raccords (E) avec une conduite d'alimentation du module (F) ou bien une conduite de retour (G), les différents raccords (E) présentant, pour les réservoirs en couches minces (B) superposés, respectivement une surface transversale augmentant en direction du flux de la conduite d'alimentation du module (F) et diminuant dans la direction du flux du retour du module (G) et les conduites d'alimentation et de retour (F, G) étant constituées respectivement d'un tube avec des pièces de branchement (H, I) pour les raccords (E), où la surface transversale de la conduite d'alimentation (F) diminue dans la direction du flux et la surface transversale de la conduite de retour (G) augmente dans la direction du flux.

2. Procédé selon la revendication 1, **caractérisé en ce que** le profil de circulation régulier de la suspension de culture conditionnée est produit en introduisant celle-ci dans un réacteur qui se compose au moins d'un module en plaques transparent, extrudé en un bloc, qui présente une multitude de canaux de passage parallèles et superposés, dont les côtés entrée et sortie débouchent dans un collecteur de fluide et/ou un répartiteur de fluides, le répartiteur de fluides vu dans la direction du flux présentant une réduction de la section transversale et le collecteur de fluide présentant une augmentation de la section transversale, moyennant quoi une vitesse de flux régulière optimale de la suspension est ajustée dans le réacteur sur tous les canaux.

3. Procédé selon les revendications 1 à 2, **caractérisé en ce que** l'on ajoute, à la suspension de culture, des sucres simples et/ ou complexes à titre d'autres sources de carbone organiques.

4. Procédé selon la revendication 3, **caractérisé en ce que** l'on ajoute, à la suspension de culture, du glucose en une concentration de 0,3 à 10 g/l de suspension de culture à titre d'autres sources de carbone organiques.

5. Procédé selon l'une des revendications précédentes, **caractérisé en ce que** la composition quantitative des ingrédients cellulaires de la biomasse est modifiée, la teneur en graisse atteignant plus de 20 % de la biomasse sèche.

6. Procédé selon l'une des revendications précédentes, **caractérisé en ce que** le produit métabolique est un gaz contenant du O₂, qui est séparé par séparation de phase après passage dans le réacteur.

7. Procédé selon l'une des revendications précédentes, **caractérisé en ce que** la séparation de la biomasse se fait en utilisant des différences de densité dans le bassin de décantation et/ ou la centrifugation et/ ou en utilisant la granulométrie dans des systèmes de filtres et **en ce que** la solution de culture est renvoyée dans le système du réacteur.

8. Procédé selon l'une des revendications précédentes, **caractérisé en ce que** l'on produit de la biomasse d'algues et leurs produits métaboliques et que l'on utilise, à titre de solution nutritive, de l'eau et des substances, qui sont nécessaires à la constitution de composés de carbone riches en énergie.

9. Procédé selon la revendication 8, **caractérisé en ce que** la solution nutritive comprend des composés azotés, des phosphates, des composés de potassium, calcium et magnésium et des éléments traces.

10. Procédé selon les revendications 8 et 9, **caractérisé en ce que** la solution nutritive comprend, à titre d'autres sources de carbone organiques, du sucre et/ou des acétates et/ou, à titre de substances tamponnantes, du tris(hydroxyméthylaminométhane) et/ ou du borax et/ou de la chaux.

11. Procédé selon les revendications 8 à 10, **caractérisé en ce que** l'on utilise, à titre de solution nutritive, des eaux usées, y compris des boues de clarification et/ou des solutions synthétiques.

12. Procédé selon l'une des revendications précédentes, **caractérisé en ce que** l'on utilise, à titre de micro-organismes, des organismes phototrophes et/ ou des cultures cellulaires.

13. Procédé selon la revendication 12, **caractérisé en ce que** l'on utilise, à titre de micro-organismes, des micro-algues.

14. Procédé selon la revendication 12 et 13, **caractérisé en ce que** l'on utilise, à titre de micro-organismes, les espèces de microalgues *Chlorella ssp.* et/ou *Scenedesmus spp.* et/ ou *Microcystis spp.* sous forme de cultures pures ou mixtes.

15. Procédé selon l'une des revendications précédentes, **caractérisé en ce que** l'on utilise un gaz de rejet à titre de gaz contenant du CO₂.

16. Procédé selon la revendication 15, **caractérisé en ce que** l'on utilise un gaz de rejet provenant de procédés de combustion, de procédés de calcination de la chaux et/ ou de procédés métallurgiques à titre de gaz contenant du CO₂.

17. Procédé selon l'une des revendications précédentes, **caractérisé en ce que** l'on tempère le gaz de rejet contenant le CO₂ dans un refroidisseur de gaz de rejet et que le condensat ainsi obtenu est amené dans le réacteur avec le gaz contenant le CO₂, par le biais d'un laveur de gaz.

18. Procédé selon l'une des revendications précédentes, **caractérisé en ce que** le gaz est amené par le laveur de gaz à contre-courant de la suspension de culture.

19. Procédé selon l'une des revendications précédentes, **caractérisé en ce que** l'éclairage de la suspension de culture se trouvant dans le réacteur se fait par rayonnement du soleil direct et/ ou diffus et/ ou éclairage artificiel.

20. Utilisation de la biomasse produite selon l'une des revendications précédentes pour la retransformation sous forme de porteurs d'énergie.

21. Utilisation de la biomasse produite selon l'une des revendications précédentes pour la fabrication de substances de base et de principes actifs chimiques et/ou pharmaceutiques.

22. Utilisation de la biomasse produite selon l'une des revendications précédentes à titre de produit alimentaire et/ ou additif alimentaire.
